# EUROPÄISCHE PATENTSCHRIFT

(11) **EP 0 842 229 B1**
(45) Veröffentlichungstag und Bekanntmachung des Hinweises auf die Patenterteilung: **06.03.2002**
(21) Anmeldenummer: 97921767.6
(22) Anmeldetag: 25.04.1997
(51) Int. Cl.: C09C 1/00, C09C 1/36, C09D 7/12, C09D 11/00, C08K 3/00, A61K 7/00

(54) **PLÄTTCHENFÖRMIGES TITANDIOXIDREDUKTIONSPIGMENT**
PLATE-LIKE TITANIUM DIOXIDE REDUCTION PIGMENT
PIGMENT DE REDUCTION DE DIOXYDE DE TITANE SOUS FORME DE PLAQUETTES

(30) Priorität: 09.05.1996 DE 19618562
(43) Veröffentlichungstag der Anmeldung: 20.05.1998
(73) Patentinhaber: MERCK PATENT GmbH, 64293 Darmstadt (DE)
(72) Erfinder: ANDES, Stephanie, D-63477 Maintal 3 (DE); HOCK, Sabine, D-64850 Schaafheim (DE); BRENNER, Günter, D-64347 Griesheim (DE); BRÜCKNER, Dieter, D-64289 Darmstadt (DE); HEYLAND, Andrea, D-64385 Ober-Kainsbach (DE); KUNTZ, Matthias, D-64342 Seeheim (DE); OSTERRIED, Karl, D-64807 Dieburg (DE); PFAFF, Gerhard, D-64839 Münster (DE); SCHMELZ, Michael, D-65830 Kriftel (DE)
(86) Internationale Anmeldenummer: EP9702133
(87) Internationale Veröffentlichungsnummer: WO9743347

(56) Entgegenhaltungen:
- EP-A- 0 338 428
- EP-A- 0 481 460
- EP-A- 0 601 761
- DE-A- 3 433 657
- US-A- 3 340 006
- US-A- 3 395 203
- DATABASE WPI Week 8345 Derwent Publications Ltd., London, GB; AN 83-810901 XP002035870 & JP 58 164 653 A (MITSUBISHI METAL)

## Beschreibung

Die Erfindung betrifft sehr dünne, plättchenförmige Titandioxidreduktionspigmente.

Plättchenförmige Titandioxidreduktionspigmente sind bekannt. Jedoch basieren sie auf der Verwendung von Glimmer als Substrat. Das Titandioxid wird auf den Glimmer aufgefällt und nachfolgend teilweise zu Titansuboxiden reduziert. Als Reduktionsmittel werden gasförmige Reduktionsmittel wie Wasserstoff und Ammoniak oder feste Reduktionsmittel wie Silicium oder Titan verwendet. Einige dieser Pigmente besitzen noch eine zusätzliche Deckschicht aus Siliciumdioxid.

US 4 948 631 beschreibt ein Verfahren zur Herstellung besonders blaustichiger Perlglanzpigmente durch Reduktion von mit Titandioxid beschichteten Glimmerpigmenten mit Ammoniak bei Temperaturen von 750 bis 850 °C.

JP H4-20 031 beschreibt ein Verfahren zur Herstellung eines farbigen Glimmerpigmentes durch Mischen eines Titandioxid-Glimmerpigmentes mit Titan und Reduzieren der erhaltenen Mischung im Vakuum bei 500 bis 1000 °C.

US 4 623 396 beschreibt ein Titandioxid-Glimmerpigment bestehend aus Glimmer, einer ersten Schicht aus Titandioxid, Titansuboxiden und Titanoxynitriden und einer zweiten Schicht aus Titandioxid.

Glimmerpigmente werden in großem Umfang in der Druck- und Lackindustrie, in der Kosmetik und in der Kunststoffverarbeitung verwendet. Sie zeichnen sich durch Interferenzfarben und einen hohen Glanz aus. Für die Ausbildung extrem dünner Schichten sind aber Glimmerpigmente nicht geeignet, weil Glimmer als Substrat für die Metalloxidschichten des Pigmentes bereits eine Dicke von 200 bis 1200 nm aufweist. Nachteilig ist weiter hin, daß die Dicke der Glimmerplättchen innerhalb einer bestimmten Fraktion, die durch die Plättchengröße bestimmt wird, teilweise deutlich um einen Mittelwert schwankt. Außerdem handelt es sich bei Glimmer um ein natürlich vorkommendes Mineral, das durch Fremdionen verunreinigt ist. Desweiteren sind technisch sehr aufwendige und zeitraubende Aufbereitungsschritte notwendig. Hierzu zählen vor allem Mahlen und Klassieren.

Auf dicken Glimmerplättchen basierende, mit Metalloxiden umhüllte Perlglanzpigmente haben aufgrund der Dicke des Randes einen deutlichen Streuanteil, speziell bei feineren Korngrößenverteilungen unter 20 µm.

Als Ersatz für Glimmer sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 µm und eine sehr breite Dickenverteilung (typischerweise zwischen 4 und 20 µm) aufweisen, während die Dicke von Interferenzpigmenten typischerweise nicht größer als 3 µm ist.

In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschließend zerkleinert und man erhält plättchenförmige Alkalisilikatsubstrate mit einer Dicke von weniger als 3 µm. Das Verfahren ist jedoch aufwendig und die Dickenverteilung der erhaltenen Plättchen ist relativ breit.

in DE 11 36 042 ist ein kontinuierliches Bandverfahren zur Herstellung plättchen- oder flitterartiger Oxide oder Oxidhydrate von Metallen der IV. und V. Gruppe sowie der Eisen-Gruppe des Periodensystems beschrieben. Dabei wird auf ein kontinuierliches Band ggf. zunächst eine Trennschicht aus z.B. Silikonlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Die Schichtdicke der erhaltenen Plättchen wird mit 0,2 bis 2 µm angegeben, ohne hierfür konkrete Beispiele zu nennen.

In EP 0,240,952 und EP 0,236,952 ist ein kontinuierliches Bandverfahren zur Herstellung verschiedener plättchenförmiger Materialien, darunter auch Siliciumdioxid, Aluminiumoxid und Titandioxid vorgeschlagen worden. Dabei wird über ein Rollensystem auf ein glattes Band ein dünner flüssiger Film definierter Dickes eines Precursors des plättchenförmigen Materials aufgebracht; der Film wird getrocknet und von dem Band abgelöst, wobei sich plättchenförmige Teilchen bilden. Die Teilchen werden anschließend gegebenenfalls geglüht, gemahlen und klassiert.

Die Dicke der nach dem in EP 0 240 952 beschriebenen Verfahren erhaltenen Plättchen ist relativ gut definiert, da der Film z.B. auf das kontinuierliche Band über ein Rollensystem sehr gleichmäßig aufgebracht wird. Die Schichtdicke der Plättchen wird in den Beispielen mit 0,3 bis 3,0 µm angegeben. Gemäß Beispiel 1 wird eine erste Rolle mit dem verwendeten Precursor benetzt, indem man diese Rolle teilweise in einen mit dem Precursor befüllten Vorratsbehälter eintaucht. Der Film wird von dieser Rolle auf eine zweite, gleichsinnig rotierende Rolle übertragen, die mit der ersten in sehr engem Kontakt steht. Schließlich wird der Film von der zweiten Rolle auf das kontinuierliche Band abgerollt.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken sich auch der hohe Energieaufwand und die Einschränkung der Prozeßgeschwindigkeit sehr nachteilig auf die Wirtschaftlichkeit des Verfahrens aus.

WO 93/08 237 beschreibt plättchenförmige Pigmente, bestehend aus einer plättchenförmigen Matrix aus Siliciumdioxid, die lösliche oder nichtlösliche Farbmittel enthalten kann und die mit einer oder mehreren reflektierenden Schichten aus Metalloxiden oder Metallen belegt ist. Die plättchenförmige Matrix wird durch Verfestigung von Wasserglas auf einem endlosen Band hergestellt.

In EP 0 481 460 werden nadelförmige oder plättchenförmige TiOₓ-Suboxide offenbart, welche zur Erhaltung ihrer ursprünglichen Abmessungen mit einem Überzug aus SiO₂ versehen sind und gute elektrische und mechanische Eigenschaften aufweisen.

In DE 1 273 098 wird die Herstellung eines Perlmuttpigmentes durch Aufdampfen von ZnS-, MgF₂-, ZnO-, CaF₂- und TiO₂-Filmen auf ein endloses Band beschrieben. Dieses Verfahren ist aber ebenso, wie das in US 4 879 140 beschriebene Verfahren, bei dem plättchenförmige Pigmente mit Si- und SiO₂-Schichten durch Plasmaabscheidung aus SiH₄ und SiCl₄ erhalten werden, mit einem sehr hohen apparativen Aufwand verbunden.

Trotz zahlreicher Versuche konnte bisher noch kein wirtschaftliches Verfahren zu Herstellung sehr dünner plättchenförmiger Titandioxidpigmente mit einer Schichtdicke von kleiner als 500 nm entwickelt werden.

Aufgabe der Erfindung ist es, ein hochglänzendes plättchenförmiges Titandioxidreduktionspigment mit einer Schichtdicke von kleiner als 500 nm und einer Schichtdickentoleranz von weniger als 10 % bereitzustellen.

Diese Aufgabe wird gelöst durch ein plättchenförmiges Titandioxidreduktionspigment bestehend aus Titandioxid, Titansuboxiden und gegebenenfalls einem weiteren Metalloxid oder Titanoxinitrid mit einer Schichtdicke von kleiner als 500 nm und einer Schichtdickentoleranz von weniger als 10 %, erhältlich durch Verfestigung eines flüssigen Precursors auf einem endlosen Band, Ablösung der entstandenen Schicht, Beschichtung der erhaltenen Titandioxidplättchen ohne Zwischentrocknung im Naßverfahren mit weiterem Titandioxid,

Trocknung und gegebenenfalls Kalzination und Behandlung des erhaltenen Materials mit einem Reduktionsmittel in einer nicht oxidierenden Gasatmosphäre.

Weiterhin wird diese Aufgabe gemäß der Erfindung gelöst durch ein Verfahren zur Herstellung der erfindungsgemäßen Pigmente, bestehend aus Titandioxid, Titansuboxiden und gegebenenfalls einem weiteren Metalloxid oder Titanoxinitrid, wobei
- ein Precursor des plättchenförmigen Titandioxides als dünner Film auf ein endloses Band aufgebracht wird,
- der flüssige Film durch Trocknung verfestigt wird und dabei das Titandioxid durch eine chemische Reaktion aus dem Precursor entwickelt wird,
- die entstandene Schicht anschließend vom Band abgelöst und gewaschen wird,
- die erhaltenen Titandioxidplättchen ohne Zwischentrocknung in Wasser suspendiert und mit weiterem Titandioxid beschichtet werden,
- die beschichteten Titandioxidplättchen aus der wäßrigen Suspension abgetrennt, getrocknet und gegebenenfalls kalziniert werden und
- mit einem Reduktionsmittel in einer nicht oxidierenden Gasatmosphäre bei erhöhten Temperaturen behandelt werden.

In einer besonderen Ausführungsform des Verfahrens werden die vom Band abgelösten Titandioxidplättchen getrocknet und unter Verwendung alternativer Verfahren, zum Beispiel CVD-Verfahren, weiter mit Titandioxid beschichtet und mit einem Reduktionsmittel in einer nicht oxidierenden Gasatmosphäre bei erhöhten Temperaturen behandelt. Unter CVD-Verfahren wird dabei die Beschichtung in der Gasphase in einem Wirbelbettreaktor verstanden, wie sie in EP 0 045 851 und WP 0 106 235 für die Herstellung von Perlglanzpigmenten beschrieben ist.

Gegenstand der Erfindung ist außerdem die Verwendung der erfindungsgemäßen Pigmente zur Pigmentierung von Lacken, Druckfarben, Kunststoffen, Kosmetika und Glasuren für Keramiken und Gläser.

Hierfür können sie auch als Mischungen mit handelsüblichen Pigmenten, beispielsweise anorganischen und organischen Absorptionspigmenten, Metallefektpigmenten und LCP-Pigmenten eingesetzt werden.

Die erfindungsgemäßen Pigmente basieren auf plättchenförmigen Titandioxidpartikeln. Diese Plättchen haben eine Dicke zwischen 10 nm und 500 nm, vorzugsweise zwischen 40 und 150 nm. Die Ausdehnung in die beiden anderen Dimensionen beträgt zwischen 2 und 200 µm und insbesondere zwischen 5 und 50 µm.

Die Titandioxidschicht, die im Naßverfahren auf die plättchenförmigen Titandioxidpartikel aufgebracht wird, besitzt eine Dicke von 5 bis 300 nm, vorzugsweise zwischen 5 und 150 nm.

Als Precursor für die Herstellung der Titandioxidplättchen auf dem endlosen Band werden wäßrige Lösungen thermisch hydrolysierbarer Titanverbindungen verwendet. Ein bevorzugter Precursor ist wäßrige Titantetrachloridlösung. Die Konzentration des Titansalzes in diesen Lösungen beträgt 7 bis 30 Gew.-%, vorzugsweise 8 bis 15 Gew.-%.

Das weitere Metalloxid, das neben Titandioxid und Titansuboxiden im erfindungsgemäßen Pigment enthalten sein kann, ist das Oxidationsprodukt des eingesetzten Reduktionsmittels. Dieses Metalloxid ist in einer Konzentration von 1 bis 20 Gew.-%, vorzugsweise 1 bis 5 Gew.-% im erfindungsgemäßen Pigment enthalten.

Als Reduktionsmittel werden gasförmige Reduktionsmittel, wie zum Beispiel Wasserstoff oder feste Reduktionsmittel in Form von Metallpulvern, Legierungen von Metallen, Metallboriden, Metallcarbiden oder Metallsiliciden eingesetzt. Bevorzugt werden Pulver der Metalle Bor, Aluminium, Silicium, Zink und Eisen und insbesondere Silicium verwendet.

Das Titandioxidpigment und das Reduktionsmittel werden in einem Verhältnis von 100:1 bis 5:1 gemischt. In einer besonderen Ausführungsform kann das erfindungsgemäße Pigment noch Ruß enthalten, der entweder direkt auf die plättchenförmigen Titandioxidpartikel aufgebracht oder zusammen mit dem Titandioxid im Naßverfahren auf die plättchenförmigen Titandioxidpartikel aufgefällt wird. Einzelheiten sind in DE 195 02 231, DE 42 22 372 und US 4 076 551 näher beschrieben.

Die Herstellung der erfindungsgemäßen Pigmente erfolgt in einem dreistufigem Verfahren. In der ersten Stufe werden plättchenförmige Titandioxidpartikel mit Hilfe eines endlosen Bandes hergestellt.

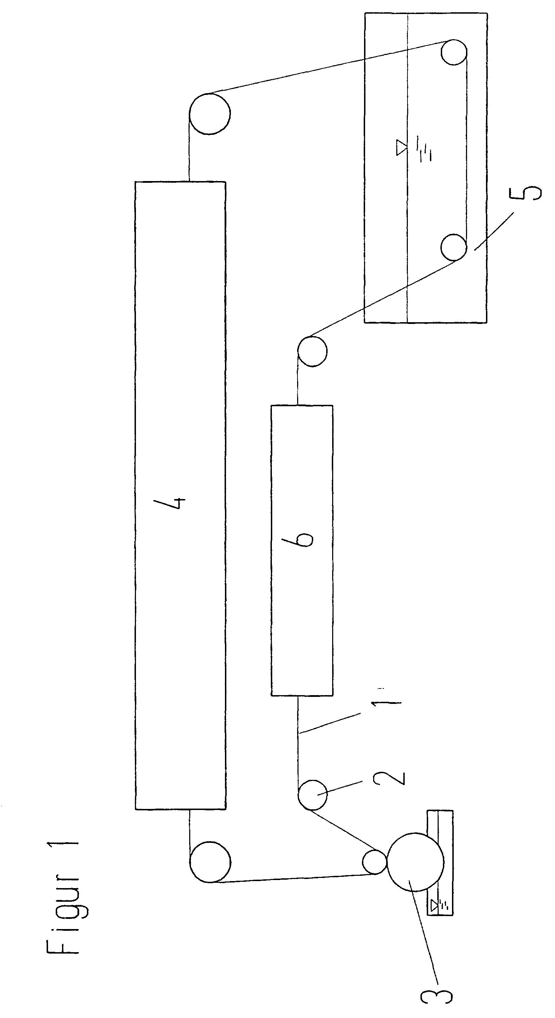

Zunächst soll an Hand von Figur 1 das Bandverfahren erläutert werden.

Das endlose Band 1, welches über ein Rollensystem 2 geführt wird, durchläuft ein Auftragswerk 3, wo es mit einem dünnen Film des Precursors beschichtet wird. Als geeignete Auftragswerke können Walzenauftragswerke sowie Fließer eingesetzt werden. Die Bandgeschwindigkeit liegt zwischen 2 und 400 m/min, vorzugsweise 5 - 200 m/min.

Um eine gleichmäßige Benetzung des Kunststoffbandes zu erzielen, ist es zweckmäßig, der Beschichtungslösung ein handelsübliches Netzmittel zuzusetzen oder die Bandoberfläche durch Beflammung, Koronabehandlung oder Ionisation zu aktivieren.

Anschließend durchläuft das beschichtete Band eine Trockenstrecke 4 in der die Schicht bei Temperaturen zwischen 30 und 200 °C getrocknet wird. Als Trockner können zum Beispiel handelsübliche Infrarot-, Umluftdüsen- und UV-Trockner eingesetzt werden.

Nach dem Passieren der Trockenstrecke wird das Band durch die Ablösebäder 5 mit einem geeignetem Ablösemedium, zum Beispiel vollentsalztem Wasser geführt, wo die getrocknete Schicht vom Band entfernt wird. Der Ablösevorgang wird hierbei durch zusätzliche Vorrichtungen, zum Beispiel Düsen, Bürsten oder Ultraschall, unterstützt.

In einem Nachtrockner 6 wird das Band vor der erneuten Beschichtung getrocknet.

Das endlose Band sollte aus einem chemisch und thermisch beständigem Kunststoff sein, um eine ausreichende Standzeit und hohe Trocknungstemperaturen zu gewährleisten. Hierfür sind Materialien wie Polyethylenterephthalat (PET) oder andere Polyester und Polyacrylate geeignet.

Die Folienbreite liegt typischerweise zwischen einigen Zentimetern bis zu mehreren Metern. Die Dicke beträgt zwischen 10 µm bis zu einigen mm, wobei diese beiden Parameter im Hinblick auf die jeweiligen Anforderungen optimiert werden.

Weitere Einzelheiten zu kontinuierlichen Bandverfahren sind aus US 3 138 475, EP 0 240 952 und WO 93/08 237 bekannt.

Die vom Band abgelösten Titandioxidplättchen werden in einer zweiten Verfahrensstufe ohne vorherige Zwischentrocknung mit weiterem Titandioxid nach bekannten Verfahren beschichtet. Bevorzugt wird das in US 3 553 001 beschriebene Verfahren verwendet.

Zu einer auf etwa 50-100 °C, insbesondere 70-80 °C erhitzten Suspension der Titandioxidplättchen wird langsam eine wäßrige Titansalzlösung zugegeben, und es wird durch gleichzeitiges Zudosieren einer Base, wie z.B. wäßrige Ammoniaklösung oder eine wäßrige Alkalilauge, ein weitgehend konstanter pH-Wert von etwa 0,5-5, insbesondere etwa 1,5-2,5 eingehalten. Sobald die gewünschte Schichtdicke der TiO₂-Fällung erreicht ist, wird die Zugabe der Titansalzlösung gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO₂ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind. Die pro Minute zugesetzte Titansalzmenge liegt dabei in der Größenordnung von etwa 0,01 bis 20·10⁻⁵ Mol Titansalz pro Quadratmeter zu belegender Oberfläche.

In einer dritten Verfahrensstufe werden die plättchenförmigen Titandioxidpartikel, die im Naßverfahren mit weiterem Titandioxid beschichtet wurden, einem Reduktionsprozeß unterzogen, wie er für die Herstellung von Titandioxidreduktionspigmenten bekannt ist. Bevorzugt wird das in WO 93/19131 beschriebene Verfahren verwendet.

Die Titandioxidpartikel werden mit den oben beschriebenen festen Reduktionsmitteln in einem Verhältnis von 100:1 bis 5:1 intensiv gemischt und bei Temperaturen von mehr als 600 °C, bevorzugt im Bereich von 700 bis 1100 °C, für mehr als 10 Minuten, bevorzugt für 15 bis 60 Minuten, in einer nichtoxidierenden Atmosphäre behandelt. Als nichtoxidierende Gase werden Stickstoff, Argon, Helium oder Kohlendioxid verwendet, wobei Stickstoff oder Argon bevorzugt werden.

Die Reduktionsreaktion wird in Gegenwart eines Halogenides beschleunigt. Bevorzugt werden Alkali- und Erdalkalimetallchloride wie z.B. Lithiumchlorid, Natriumchlorid, Kaliumchlorid, Magnesiumchlorid und Calciumchlorid oder andere Metallchloride wie z.B. Eisen II- und Eisen III-chlorid, Nickelchlorid, Kupferchlorid, Manganchlorid und Chromchlorid verwendet. Besonders bevorzugt wird Calciumchlorid eingesetzt. Bezogen auf das eingesetzte Titandioxidpigment werden 0,1 bis 40 Gew.-%, bevorzugt 0,5 bis 10 Gew.-% Chlorid eingesetzt.

Als gasförmige Reduktionsmittel können Wasserstoff-Stickstoff-Gemische oder Ammoniak verwendet werden. Bevorzugt wird ein Wasserstoff-Stickstoff-Gemisch mit einem Wasserstoffanteil von 3 Vol.-% verwendet

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Turnbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metalchalkogenidhydrate und Ruß für eine zusätzliche Beschichtung eingesetzt werden.

Es ist weiterhin möglich, die Pigmente einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE PS 22 15 191, DE OS 31 51 354, DE OS 32 35 017 oder DE OS 33 34 598 beschriebenen Verfahren in Frage. Aufgrund der bereits ohne diese zusätzlichen Maßnahmen sehr guten Eigenschaften der erfindungsgemäßen Pigmente machen diese ggf. noch aufgebrachten Stoffe nur etwa 0 bis 5, insbesondere etwa 0 bis 3 Gew.% des gesamten Pigments aus.

Das erfindungsgemäße Pigment zeichnet sich gegenüber den bisher bekannten Titandioxidreduktionspigmenten durch einen höheren Glanz und eine sehr gleichmäßige Dicke aus. Die als Dickentoleranz bezeichnete Standardabweichung ist nicht größer als 10 %. Durch die planparallele Oberfläche und die enge Dickentoleranz der Pigmentteilchen wird eine sehr große Farbreinheit und sehr hohe Farbstärke erreicht.

Das erfindungsgemäße Pigment stellt bezüglich der Dicke den Idealzustand dar, der bei Perlglanzpigmenten maximal erreichbar ist, da es nur aus optisch funktionellen Schichten besteht und ein sonst übliches Trägermaterial, wie beispielsweise Glimmer oder Glasplättchen, das nicht zum optischen Effekt beiträgt, fehlt. Bedingt durch die Dicke des Glimmers besitzen Glimmerpigmente eine bis um den Faktor 25 größere Dicke bei gleicher Dicke der funktionellen Schichten. Bezüglich der technischen Anwendungen ergeben sich hieraus intrinsische Vorteile, die von keinem anderen konventionellen Perlglanzpigment erreicht werden können. Beispielsweise können Lackschichten dünner ausgeführt werden und die notwendige Pigmentmenge kann reduziert werden, weil aufgrund des Fehlens des "Füllstoffs" Trägermaterial die Pigmente optisch aktiver sind.

Die im folgenden angegebenen Beispielen sollen die Erfindung erläutern, ohne sie zu begrenzen.

### Beispiel 1

Ein umlaufendes Band aus Polyethylenterephthalat (Breite: 0,3m, Geschwindigkeit: 20 m/min) wird über eine Auftragswalze im Gegenlauf mit einer 20%iger Titantetrachloridlösung beschichtet. Die Beschichtungslösung enthält 0,3 Gew.-% Tensid (DISPERSE-AYD W-28, Hersteller: DANIEL PRODUCTS COMPANY). Der auf dem Band befindliche wäßrige Film wird in einer Trocknungsstrecke durch Beaufschlagung mit Heißluft von 70 °C getrocknet und die gebildete Schicht in einem mit vollentsalztem Wasser gefüllten Ablösebecken vom Band abgelöst. Die plättchenförmigen Titandioxidpartikel werden filtriert und mit vollentsalztem Wasser gewaschen. Die silbrig glänzenden Plättchen haben eine Schichtdicke von 100 ± 10 nm. Zur Beschichtung mit weiterem Titandioxid werden sie in vollentsalztem Wasser redispergiert.

2 I der Dispersion (Trockensubstanzgehalt: 15 g TiO₂) werden auf 75 °C erhitzt und mit verdünnter Salzsäure auf einen pH-Wert von 1,8 eingestellt.

4,3 g SnCl₄ · 5 H₂O werden in 29 ml HCI gelöst, mit destilliertem Wasser auf 290 ml aufgefüllt und 10 min gerührt.

Die SnCl₄-Lösung wird mit 3 ml/min zur TiO₂-Suspension zugegeben und dabei der pH 1,8 mit 32%iger NaOH-Lösung konstant gehalten. Nach der SnO₂-Belegung wird die Suspension 15 min bei konstanter Temperatur und konstantem pH-Wert nachgerührt.

Ein 40%iger wäßrige Titantetrachloridlösung wird nun mit 3 ml/min zudosiert und der pH-Wert weiterhin mit 32%iger NaOH konstant auf 1,8 gehalten.

Die TiCl₄-Zugabe wird fortgesetzt bis die gewünschte Interferenzfarbe erster oder höherer Ordnung erreicht ist. Das erhaltene Pigment wird abfiltriert, mit VE-Wasser salzfrei gewaschen, getrocknet und bei 750 °C geglüht. Die koloristischen Eigenschaften ändern sich in Abhängigkeit von der Glühtemperatur nur wenig und man erhält in allen Fällen Perlglanzpigmente mit Rutil-Struktur.

10 g Titandioxid-Pigment mit roter Interferenzfarbe werden mit 0,2 g Silicium-Pulver (Partikelgröße: < 150 µm, Hersteller: Merck KGaA) intensiv vermischt und in einem Röhrenofen unter Stickstoff bei 850 °C für einen Zeitraum von 45 Min. geglüht.

Man erhält ein Pigment mit schwarzroter Pulverfarbe und leuchtendroter Interferenzfarbe.

### Beispiel 2

2 I der Dispersion von TiO₂-Plättchen (Trockensubstanzgehalt: 15 g TiO₂) aus Beispiel 1 werden auf 75 °C erhitzt und mit verdünnter Salzsäure auf einen pH-Wert von 2,2 eingestellt.

Eine 40%ige wäßrige Titantetrachloridlösung wird nun mit 3 ml/min zudosiert und der pH-Wert weiterhin mit 32%iger NaOH konstant auf 2,2 gehalten.

Die TiCl₄-Zugabe wird fortgesetzt bis die gewünschte Interferenzfarbe erster oder höherer Ordnung erreicht ist. Das erhaltene Pigment wird abfiltriert, mit VE-Wasser salzfrei gewaschen, getrocknet und bei 750 °C geglüht. Die koloristischen Eigenschaften ändern sich in Abhängigkeit von der Glühtemperatur wesentlich stärker als in Beispiel 1, bedingt durch die Anatas-Rutil-Umwandlung, die bei ca. 600 °C beginnt und bei 750 °C abgeschlossen ist. Es ist somit möglich TiO₂-Perlglanzpigmente Sn-frei in der Rutil-Modifikation herzustellen.

10 g Titandioxid-Pigment mit roter Interferenzfarbe werden mit 0,2 g Silicium-Pulver (Partikelgröße: < 150 µm, Hersteller: Merck KGaA) intensiv vermischt und in einem Röhrenofen unter Stickstoff bei 850 °C für einen Zeitraum von 45 Min. geglüht.

Man erhält ein Pigment mit dunkelroter Pulverfarbe und schwarzroter Interferenzfarbe.

### Beispiel 3

2 I der Dispersion von TiO₂-Plättchen (Trockensubstanzgehalt: 15 g TiO₂) aus Beispiel 1 werden auf 75 °C erhitzt und mit verdünnter Salzsäure auf einen pH-Wert von 2,2 eingestellt.

Eine 40%ige wäßrige Titantetrachloridlösung wird nun mit 3 ml/min zudosiert und der pH-Wert weiterhin mit 32%iger NaOH konstant auf 2,2 gehalten.

Die TiCl₄-Zugabe wird fortgesetzt bis die gewünschte Interferenzfarbe erster oder höherer Ordnung erreicht ist. Das erhaltene Pigment wird abfiltriert, mit VE-Wasser salzfrei gewaschen, getrocknet und bei 750 °C geglüht. Die koloristischen Eigenschaften ändern sich in Abhängigkeit von der Glühtemperatur wesentlich stärker als in Beispiel 1, bedingt durch die Anatas-Rutil-Umwandlung, die bei ca. 600 °C beginnt und bei 750 °C abgeschlossen ist. Es ist somit möglich TiO₂-Perlglanzpigmente Sn-frei in der Rutil-Modifikation herzustellen.

10 g Titandioxid-Pigment mit roter Interferenzfarbe werden in einem Röhrenofen unter Stickstoff bei 850 °C für einen Zeitraum von 45 min in einer Stickstoff-Wasserstoff-Atmosphäre mit einem Volumenanteil an Wasserstoff von 3 % geglüht.

Man erhält ein Pigment mit dunkelroter Pulverfarbe und schwarzroter Interferenzfarbe.

## Patentansprüche

1. Plättchenförmiges Titandioxidreduktionspigment bestehend aus Titandioxid, Titansuboxiden und gegebenenfalls einem weiteren Metalloxid oder Titanoxidnitrid mit einer Schichtdicke von kleiner als 500 nm und einer Schichtdickentoleranz von weniger als 10%, erhältlich durch Verfestigung einer wäßrigen Lösung einer thermisch hydrolisierbaren Titanverbindung auf einem endlosen Band, Ablösung der entstandenen Schicht, Beschichtung der erhaltenen Titandioxidplättchen ohne Zwischentrocknung im Naßverfahren mit weiterem Titandioxid, Trocknung und gegebenenfalls Kalzination und Behandlung des erhaltenen Materials mit einem Reduktionsmittel in einer nicht oxidierenden Gasatmosphäre.

2. Titandioxidreduktionspigment nach Anspruch 1, **dadurch gekennzeichnet, daß** das weitere Metalloxid Li₂O, Na₂O, K₂O, MgO, CaO, B₂O₃, Al₂O₃, SiO₂, ZnO, SnO₂ oder Fe₂O₃ ist.

3. Titandioxidreduktionspigment nach den Ansprüchen 1 und 2, **dadurch gekennzeichnet, daß** die wäßrige Lösung einer thermisch hydrolisierbaren Titanverbindung eine wäßrige Titantetrachloridlösung ist.

4. Verfahren zur Herstellung des Titandioxidreduktionspigmentes nach den Ansprüchen 1 bis 3, **dadurch gekennzeichnet, daß**
- eine wäßrige Lösung einer thermisch hydrolysierbaren Titanverbindung als dünner Film auf ein endloses Band aufgebracht wird,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidplättchen ohne Zwischentrocknung in Wasser suspendiert und mit weiteren Titandioxid beschichtet werden,
- die Titandioxidplättchen aus der wäßrigen Suspension abgetrennt, getrocknet und gegebenenfalls kalziniert werden und
- mit einem Reduktionsmittel in einer nicht oxidierenden Gasatmosphäre bei erhöhten Temperaturen behandelt werden.

5. Verfahren nach Anspruch 4, **dadurch gekennzeichnet, daß** die wäßrige Lösung einer thermisch hydrolisierbaren Titanverbindung eine wäßrige Titantetrachloridlösung ist.

6. Verfahren nach den Ansprüchen 4 und 5, **dadurch gekennzeichnet, daß** das Reduktionsmittel ein Alkalimetall, B, Al, Si, Zn, Fe, LiH, CaH₂, Al₄C₃, Mg₂Si, MgSi₂ oder CaSi₂ ist.

7. Verfahren nach mindestens einem der Ansprüche 4 bis 6, **dadurch gekennzeichnet, daß** das Reduktionsmittel Ammoniak oder ein Wasserstoff-Stickstoff-Gemisch ist.

8. Verfahren nach mindestens einem der Ansprüche 4 bis 7, **dadurch gekennzeichnet, daß** das weitere Titandioxid in einem Wirbelbettreaktor durch CVD auf die Titandioxidplättchen aufgebracht wird.

9. Verwendung der Pigmente gemäß den Ansprüchen 1 bis 3 zur Pigmentierung von Lacken, Druckfarben, Kunststoffen, Kosmetika und Glasuren für Keramiken und Gläser.

10. Verwendung nach Anspruch 9, **dadurch gekennzeichnet, daß** die Pigmente als Mischungen mit handelsüblichen Pigmenten eingesetzt werden.

11. Lacke, Druckfarben, Kunststoffe, Kosmetika, Keramiken und Gläser, welche mit einem Pigment nach den Ansprüchen 1 bis 3 pigmentiert sind.

## Claims

1. Plateletlike titanium dioxide reduction pigment consisting of titanium dioxide, titanium suboxides and, if desired, a further metal oxide or titanium oxynitride having a layer thickness of less than 500 nm and a layer thickness tolerance of less than 10%, obtainable by solidifying an aqueous solution of a thermally hydrolysable titanium compound on a continuous belt, detaching the resulting layer, coating the resulting titanium dioxide platelets, without drying in between, with further titanium dioxide by a wet method, drying and, if desired, calcining the material, and treating the material obtained with a reducing agent in a nonoxidizing gas atmosphere.

2. Titanium dioxide reduction pigment according to Claim 1, **characterized in that** the further metal oxide is Li₂O, Na₂O, K₂O, MgO, CaO, B₂O₃, Al₂O₃, SiO₂, ZnO, SnO₂ or Fe₂O₃.

3. Titanium dioxide reduction pigment according to Claims 1 and 2, **characterized in that** the aqueous solution of a thermally hydrolysable titanium compound is an aqueous titanium tetrachloride solution.

4. Process for the preparation of the titanium dioxide reduction pigment according to Claims 1 to 3, **characterized in that**
- an aqueous solution of a thermally hydrolysable titanium compound is applied as a thin film to a continuous belt
- the liquid film is solidified by drying, during the course of which the titanium dioxide is developed from the solution by means of a chemical reaction,
- the resulting layer is subsequently detached from the belt and washed,
- the titanium dioxide platelets obtained, without drying in between, are suspended in water and coated with further titanium dioxide,
- the titanium dioxide platelets are separated out from the aqueous suspension, dried and, if desired, calcined, and
- are treated with a reducing agent in a nonoxidizing gas atmosphere at elevated temperatures.

5. Process according to Claim 4, **characterized in that** the aqueous solution of a thermally hydrolysable titanium compound is an aqueous titanium tetrachloride solution.

6. Process according to Claims 4 and 5, **characterized in that** the reducing agent is an alkali metal, B, Al, Si, Zn, Fe, LiH, CaH₂, Al₄C₃, Mg₂Si, MgSi₂ or CaSi₂.

7. Process according to at least one of Claims 4 to 6, **characterized in that** the reducing agent is ammonia or a hydrogen-nitrogen mixture.

8. Process according to at least one of Claims 4 to 7, **characterized in that** the further titanium dioxide is applied to the titanium dioxide platelets in a fluidized-bed reactor by CVD.

9. Use of the pigments according to Claims 1 to 3 for pigmenting paints, printing inks, plastics, cosmetics and glazes for ceramics and glass.

10. Use according to Claim 9, **characterized in that** the pigments are employed as mixtures with commercially available pigments.

11. Paints, printing inks, plastics, cosmetics, ceramics and glass which are pigmented with a pigment according to Claims 1 to 3.

## Revendications

1. Pigment en paillettes réduit à base de dioxyde de titane, constitué de dioxyde de titane, de sous-oxydes de titane et éventuellement d'un autre oxyde métallique ou de nitrure-oxyde de titane, ayant une épaisseur de couche inférieure à 500 nm et une tolérance d'épaisseur de couche de moins de 10 %, pouvant être obtenu par solidification d'une solution aqueuse d'un composé contenant du titane, hydrolysable à la chaleur, sur une bande continue, détachement de la couche formée, enrobage avec une nouvelle quantité de dioxyde de titane des paillettes de dioxyde de titane obtenues, sans séchage intermédiaire, dans le procédé en humide, séchage et éventuellement calcination et traitement du produit obtenu par un réducteur dans une atmosphère gazeuse non oxydante.

2. Pigment réduit à base de dioxyde de titane selon la revendication 1, **caractérisé en ce que** l'autre oxyde métallique est Li₂O, Na₂O, K₂O, MgO, CaO, B₂O₃, Al₂O₃, SiO₂, ZnO, SnO₂ ou Fe₂O₃.

3. Pigment réduit à base de dioxyde de titane selon les revendications 1 et 2, **caractérisé en ce que** la solution aqueuse d'un composé contenant du titane, hydrolysable à la chaleur, est une solution aqueuse de tétrachlorure de titane.

4. Procédé pour la préparation du pigment réduit à base de dioxyde de titane selon les revendications 1 à 3, **caractérisé en ce que**
- on applique sur une bande continue une solution aqueuse d'un composé contenant du titane, hydrolysable à la chaleur, sous forme d'une mince pellicule,
- la mince pellicule est solidifiée par séchage et pendant ce séchage le dioxyde de titane se développe par une réaction chimique à partir de la solution,
- la couche formée est ensuite détachée de la bande et lavée,
- les paillettes de dioxyde de titane obtenues sont mises en suspension dans de l'eau, sans séchage intermédiaire, et enrobées avec une nouvelle quantité de dioxyde de titane,
- les paillettes de dioxyde de titane sont séparées de la suspension aqueuse, séchées et éventuellement calcinées et
- sont traitées par un réducteur dans une atmosphère gazeuse non oxydante, à hautes températures.

5. Procédé selon la revendication 4, **caractérisé en ce que** la solution aqueuse d'un composé contenant du titane, hydrolysable à la chaleur, est une solution aqueuse de tétrachlorure de titane.

6. Procédé selon les revendications 4 et 5, **caractérisé en ce que** le réducteur est un métal alcalin, B, Al, Si, Zn, Fe, LiH, CaH₂, Al₄C₃, Mg₂Si, MgSi₂ ou CaSi₂.

7. Procédé selon au moins l'une des revendications 4 à 6, **caractérisé en ce que** le réducteur est l'ammoniac ou un mélange d'hydrogène et d'azote.

8. Procédé selon au moins l'une des revendications 4 à 7, **caractérisé en ce que** la nouvelle quantité de dioxyde de titane est appliquée sur les paillettes de dioxyde de titane, par CVD (dépôt chimique en phase vapeur) dans un réacteur à lit fluidisé.

9. Utilisation des pigments selon les revendications 1 à 3, pour la pigmentation de peintures, d'encres d'imprimerie, de matières plastiques, de cosmétiques et de glaçures pour céramiques et verres.

10. Utilisation selon la revendication 9, **caractérisée en ce que** les pigments sont utilisés sous forme de mélanges avec des pigments du commerce.

11. Peintures, encres d'imprimerie, matières plastiques, cosmétiques, céramiques et verres, qui sont pigmentés avec un pigment selon les revendications 1 à 3.
